# EUROPÄISCHE PATENTSCHRIFT

(11) **EP 1 675 514 B1**
(45) Veröffentlichungstag und Bekanntmachung des Hinweises auf die Patenterteilung: **31.03.2010**
(21) Anmeldenummer: 03750230.9
(22) Anmeldetag: 21.10.2003
(51) Int. Cl.: A61B 17/72

(54) **MARKNAGEL**
INTRAMEDULLARY NAIL
CLOU INTRAMEDULLAIRE

(43) Veröffentlichungstag der Anmeldung: 05.07.2006
(73) Patentinhaber: Synthes GmbH, 4436 Oberdorf (CH)
(72) Erfinder: SCHLIENGER André, CH-4142 Münchenstein (CH); BUETTLER, Markus, CH-4717 Mümliswil (CH); SENN, Peter, CH-4437 Waldenburg (CH)
(74) Vertreter: Lusuardi, Werther
(86) Internationale Anmeldenummer: PCT/CH2003/000683
(87) Internationale Veröffentlichungsnummer: WO 2005/037116

(56) Entgegenhaltungen:
- EP-A- 0 381 462
- EP-A- 1 260 188
- CH-A- 674 613
- US-A- 5 573 536
- US-A- 6 010 506
- US-A1- 2003 069 581

## Beschreibung

Die Erfindung bezieht sich auf einen Marknagel, insbesondere für die Tibia gemäss dem Oberbegriff des Patentanspruchs 1.

Aus der CH-A 674 613 (Basis für den Oberbegriff des Anspruchs 1) ist ein gattungsgemässer Marknagel bekannt, der ein proximales und ein distales Endteil aufweist, welche beide vom Mittelteil abgewinkelt sind. Das proximale Endteil kann eine Krümmung mit einem maximalen Radius von 220 mm aufweisen.

Da die Tibiae naturgemäss bei jedem Patienten unterschiedlich ausgeprägt sind und im speziellen eine unterschiedliche Länge und Tibiaplateau-Grösse aufweisen - welche voneinander abhängig sind - sollte auch der Marknagel je nach Länge veränderliche Parameter aufweisen. Ein fester für alle Marknagellängen gültiger Krümmungsradius ist somit nicht optimal für die Insertion, weil dies zu einem erhöhten Kraftaufwand und zu einem erhöhten Verlust der Reposition führt.

Hier will die Erfindung Abhilfe schaffen. Der Erfindung liegt die Aufgabe zugrunde, einen Marknagel zu schaffen, welcher die anatomischen Verhältnisse der Tibia - relativ zu ihrer Länge - berücksichtigt und insbesondere zu ihrem Markkanalverlauf optimiert ist.

Die Erfindung löst die gestellte Aufgabe mit einem Marknagel, welcher die Merkmale des Anspruchs 1 aufweist.

Die durch die Erfindung erreichten Vorteile sind im wesentlichen darin zu sehen, dass dank des erfindungsgemässen Marknagels:
a) die Insertionskraft bei gewissen Indikationen - insbesondere bei der unaufgebohrten Technik - reduziert ist,
b) durch die geringeren Insertionskräfte ergibt sich ein geringerer Repositionsverlust;
c) Der Marknagel liegt nach erfolgter Insertion in biomechanisch idealer Lage im Markkanal.
d) Sobald der Marknagel bei der Insertion an der posterioren Wand anstösst wird seine Krümmung wirksam (beim Stand der Technik muss der Marknagel an diesem Punkt entweder verbogen werden oder es muss der Verlust der Reposition in Kauf genommen werden).

Das distale Endteil des Marknagels ist als gerader Abschnitt mit der Länge "I" ≤ L ausgebildet. Dadurch ergeben sich verschiedene Vorteile, nämlich:
a) eine Übereinstimmung mit der biomechanischen Achse;
b) die Möglichkeit einer distalen Frakturversorgung ohne Repositionsverlust; und
c) die Vermeidung der Verschiebung von distalen Knochenfragmenten.

Die Länge "I" des distalen Endteils des Marknagels liegt zweckmässigerweise im Bereich von 0,20 L bis 0,55 L , vorzugsweise im Bereich von 0,25 L bis 0,50 L.

Bei einer besonderen Ausführungsform schliesst der gekrümmte Abschnitt mit dem geraden Abschnitt des Marknagels einen Winkel alpha ein, der im Bereich von 7° - 12 ° und vorzugsweise im Bereich von 8°- 9° liegt. Zusammen mit dem distalen Endteil und dem speziellen Krümmungsradius ergibt sich eine - relativ zum Eintrittspunkt des Marknagels - optimale Lage des Marknagels im Markkanal.

Bei einer besonderen Ausführungsform ist der orthogonal zur Zentralachse stehende Querschnitt des Marknagels unrund und vorzugsweise oval oder elliptisch ausgebildet. Dadurch lässt sich der Marknagel tiefer in den Markkanal einbringen, bis er an der posterioren Wand ansteht.

Bei einer weiteren Ausführungsform weist der Marknagel eine zur Zentralachse koaxiale Längsbohrung (7) auf. Dies ermöglicht eine Einführung des Marknagels über einen Führungsdraht, welcher auch für die aufgebohrte Operationstechnik verwendet werden kann.

Bei einer weiteren Ausführungsform ist das proximale Endteil des Marknagels als gerader Abschnitt der Länge P ≤ L ausgebildet. Dies bringt produktionstechnische Vorteile. Die Länge P des proximalen Endteils liegt zweckmässigerweise im Bereich von 1/6 L bis 1/3 L.

Bei einer weiteren Ausführungsform ist im Bereich des proximalen Endteils mindestens ein quer zur Zentralachse verlaufendes Verriegelungsloch vorhanden.

Bei einer weiteren Ausführungsform ist Bereich des distalen Endteils mindestens ein quer zur Zentralachse verlaufendes Verriegelungsloch vorhanden. Bei Verwendung eines, durch das Verriegelungsloch eingeführten, Verriegelungselementes wird sowohl eine Positionsfixierung in axialer Richtung, d.h. Fixierung gegen axiale Dislokation, als auch eine Rotationsfixierung, d.h. Fixierung gegen torsionale Dislokation, bewirkt.

Bei einer weiteren liegt der Krümmungsradius R des gekrümmten Abschnitts im Bereich von 350 bis 1200 mm, vorzugsweise im Bereich von 400 bis 1100 mm. Das Verhältnis UR liegt zweckmässigerweise im Bereich von 0,3 bis 0,7 , vorzugsweise im Bereich von 0,4 bis 0,6.

Bei einer weiteren Ausführungsform sind im Bereich des distalen Endteils zwei quer zur Zentralachse verlaufende Verriegelungslöcher vorhanden. Dadurch ergeben sich mehrere Vorteile:
a) die Möglichkeit in mehreren Richtungen zu Verriegeln;
b) eine winkelstabilere Fixierung der Knochenfragmente zueinander;
c) eine günstigere Aufnahme von Biegemomenten und axialen oder torsionalen Lasten.

Bei einer weiteren Ausführungsform schliessen die beiden quer zur Zentralachse verlaufenden Verriegelungslöcher untereinander einen Winkel von 45° bis 90° Grad ein.

Bei einer weiteren Ausführungsform weist das distale Endteil drei Verriegelungslöcher auf, wobei das mittlere Verriegelungsloch zu den beiden anderen Verriegelungslöchern eine unterschiedlich Distanz aufweist. Der Vorteil dieser Anordnung liegt darin, dass keine der Achsen der drei Verriegelungslöcher als potentielle Rotationsachse dienen kann. Dies führt zu einem reduzierten Spiel zwischen dem Marknagel und den Verriegelungselementen.

Im folgenden wird das Implantationsverfahren anhand eines kannulierten Marknagels kurz beschrieben:
Schritt A: Erstellen und Halten der optimalen Reposition abhängig vom Frakturtyp
Schritt B: Eröffnung des Markkanals mit Hilfe eines Eröffnungsinstrumentariums, so dass der Eintrittswinkel und die Orientierung zum Markkanal - gemäss der verwendeten Operationstechnik - stimmt.
Schritt C: Einführung eines Führungsdrahtes bis in die distale, zukünftige Endlage des Marknagels und Bestimmung der Länge des erforderlichen Marknagels;
Schritt D: Der auf dem Insertionshandgriff vormontierte Markangel wird über dem Führungsdraht durch den Eröffnungskanal in den Markraum eingebracht
Schritt E: Nach Kontrolle der axialen Lage des Marknagels und der Reposition wird der Marknagel über seine Verriegelungsoptionen verriegelt.

Die Erfindung und Weiterbildungen der Erfindung werden im folgenden anhand der teilweise schematischen Darstellungen eines Ausführungsbeispiels noch näher erläutert.

Es zeigen:
- Fig. 1: einen Längsschnitt durch den Marknagel; und
- Fig. 2: einen Schnitt längs der Linie II-II in Fig. 1.

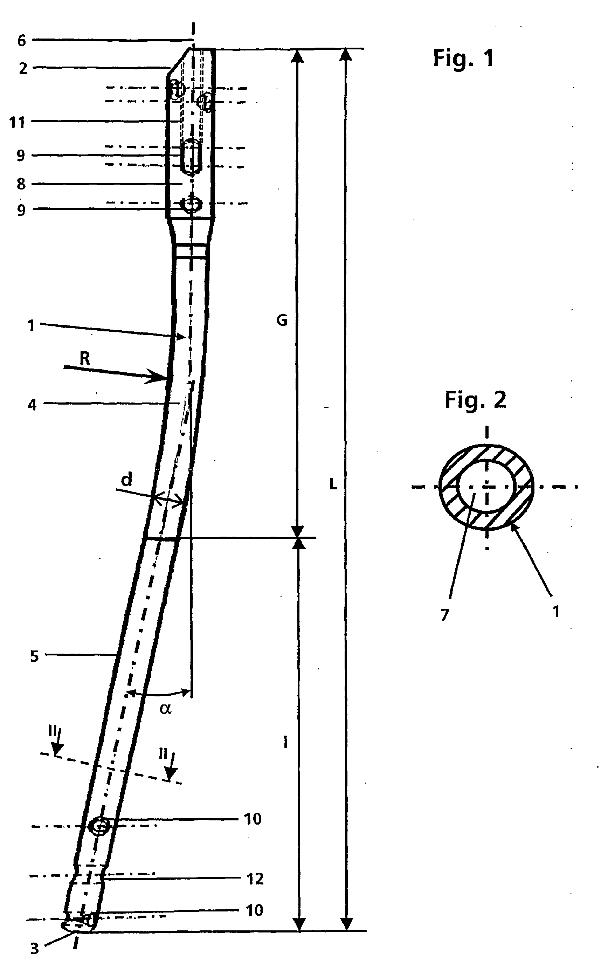

Der in den Fig. 1 und 2 dargestellte Marknagel 1 ist für eine Anwendung an der Tibia vorgesehen. Es besitzt ein proximales Endteil 2, ein distales zur Einführung in den Markraum geeignetes Endteil 3 und eine Zentralachse 6. Das proximale Endteil 2 ist mit einer Gewindebohrung 11 versehen, um eine übliche Zielhilfe aufnehmen zu können. Die Gesamtlänge L des Marknagels 1 beträgt 255 mm. Im weiteren besitzt der Marknagel einen gekrümmten Abschnitt 4 der Länge G = 127,5 mm, welcher einen Krümmungsradius R von 380 mm besitzt. Das Verhältnis UR beträgt somit 0,67. Die Krümmung des Marknagels in der Zeichenebene entspricht - nach erfolgter Implantation des Marknagels 1 - der anatomischen medio-lateralen Ebene, d.h. der Marknagel 1 ist nach erfolgter Implantation in antero-posteriorer Richtung gebogen.

Das distale Endteil 3 ist als gerader Abschnitt 5 mit der Länge "I" = 127,5 mm ausgebildet. Der gekrümmte Abschnitt 4 schliesst mit dem geraden Abschnitt 5 einen Winkel alpha von 8° ein.

Im weiteren weist der Marknagel eine zur Zentralachse 6 koaxiale, durchgehende Längsbohrung 7 auf.
Das proximale Endteil 2 ist als gerader Abschnitt 8 der Länge P = 75 mm ausgebildet, so dass P = 0,3 L beträgt.

Im Bereich des proximalen Endteils 2 des Marknagels 1 sind zwei quer zur Zentralachse 6 verlaufende Verriegelungslöcher 9 vorhanden, wobei eines davon als Langloch ausgebildet ist, um eine Kompression durchführen zu können.

Im Bereich des distalen Endteils 3 sind drei quer zur Zentralachse 6 verlaufende Verriegelungslöcher (10,12,10) vorhanden, welche in unterschiedlichen radialen Richtungen angeordnet sind und untereinander einen Winkel von 90° Grad einschliessen. Dabei weist das mittlere Verriegelungsloch 12 zu den beiden anderen Verriegelungslöchern 10 eine unterschiedlich Distanz auf.

## Patentansprüche

1. Marknagel (1), insbesondere für die Tibia, mit einem proximalen Endteil (2), einem distalen zur Einführung in den_Markraum geeigneten Endteil (3) und einer Zentralachse (6), der
A) eine Gesamtlänge L im Bereich von 200 bis 500 mm aufweist; und
B) einen gekrümmten Abschnitt (4) der Länge G ≤ L aufweist;
**dadurch gekennzeichnet, dass**
C) der gekrümmte Abschnitt (4) der Länge G einen Krümmungsradius R im Bereich von 300 - 1300 mm aufweist;
D) das Verhältnis UR im Bereich von 0,2 bis 0,8 liegt; und
E) das distale Endteil (3) als gerader Abschnitt (5) mit der Länge "I" ≤ L ausgebildet ist.

2. Marknagel (1) nach Anspruch 1, **dadurch gekennzeichnet, dass** die Länge "I" im Bereich von 0,20 L bis 0,55 L liegt.

3. Marknagel (1) nach Anspruch 2, **dadurch gekennzeichnet, dass** die Länge "I" im Bereich von 0,25 L bis 0,50 L liegt.

4. Marknagel (1) nach Anspruch 2 oder 3, **dadurch gekennzeichnet, dass** die Tangenten in den beiden Endpunkten des gekrümmten Abschnitts (4) einen Winkel alpha im Bereich von 7° -12 °, vorzugsweise 8° - 10° einschliessen.

5. Marknagel (1) nach einem der Ansprüche 1 bis 4, **dadurch gekennzeichnet, dass** der orthogonal zur Zentralachse (6) stehenden Querschnitt unrund und vorzugsweise oval oder elliptisch ausgebildet ist.

6. Marknagel (1) nach einem der Ansprüche 1 bis 5, **dadurch gekennzeichnet, dass** er zur Zentralachse (6) koaxiale Längsbohrung (7) aufweist

7. Marknagel (1) nach einem der Ansprüche 1 bis 6, **dadurch gekennzeichnet, dass** das proximale Endteil (2) als gerader Abschnitt (8) der Länge P ≤ L ausgebildet ist.

8. Marknagel (1) nach Anspruch 7, **dadurch gekennzeichnet, dass** die Länge P des proximalen Endteils (2) im Bereich von 1/6 L bis 1/3 L liegt.

9. Marknagel (1) nach einem der Ansprüche 1 bis 8, **dadurch gekennzeichnet, dass** im Bereich des proximalen Endteils (2) mindestens ein quer zur Zentralachse (6) verlaufendes Verriegelungsloch (9) vorhanden ist.

10. Marknagel (1) nach einem der Ansprüche 1 bis 9, **dadurch gekennzeichnet, dass** im Bereich des distalen Endteils (3) mindestens ein quer zur Zentralachse (6) verlaufendes Verriegelungsloch (10) vorhanden ist

11. Marknagel (1) nach einem der Ansprüche 1 bis 10, **dadurch gekennzeichnet, dass** der Krümmungsradius R des gekrümmten Abschnitts (4) im Bereich von 350 bis 1200 mm, vorzugsweise im Bereich von 400 bis 1100 mm liegt

12. Marknagel (1) nach einem der Ansprüche 1 bis 11, **dadurch gekennzeichnet, dass** das Verhältnis L/R im Bereich von 0,3 bis 0,7 ,vorzugsweise von 0,4 bis 0,6 liegt.

13. Marknagel (1) nach einem der Ansprüche 1 bis 12, **dadurch gekennzeichnet, dass** im Bereich des distalen Endteils (3) zwei quer zur Zentralachse (6) verlaufende Verriegelungslöcher (10) vorhanden sind.

14. Marknagel (1) nach Anspruch 13, **dadurch gekennzeichnet, dass** die beiden quer zur Zentralachse (6) verlaufenden Verriegelungslöcher (10) untereinander einen Winkel von 45° bis 90° Grad einschliessen.

15. Marknagel (1) nach einem der Ansprüche 1 bis 14, **dadurch gekennzeichnet, dass** das distale Endteil (3) drei Verriegelungslöcher (10,12,10) aufweist, wobei das mittlere Verriegelungsloch (12) zu den beiden anderen Verriegelungslöchern (10) eine unterschiedlich Distanz aufweist.

## Claims

1. Intramedullary nail (1), in particular for the tibia, with a proximal extremity (2), a distal extremity (3) suitable for inserting into the medullary space and a central axis (6), which
A) presents an overall length L in the range from 200 to 500 mm; and
B) presents a curved section (4) of a length G ≤ L;
**characterized in that**
C) the curved section (4) of the length G presents a radius of curvature R in the range from 300 to 1300 mm,
D) the ratio UR is in the range from 0.2 to 0.8; and
E) the distal extremity (3) is conformed as a straight section of a length "l" ≤ L.

2. Intramedullary nail (1) according to claim 1, **characterized in that** the length "I" is in the range from 0.20 L to 0.55 L.

3. Intramedullary nail (1) according to claim 2, **characterized in that** the length "I" is in the range from 0.25 L to 0.50 L.

4. Intramedullary nail (1) according to claim 2 or 3, **characterized in that** the tangents to the two endpoints of the curved section (4) enclose an angle alpha in the range from 7° to 12°, preferably from 8° to 10°.

5. Intramedullary nail (1) according to one of the claims from 1 to 4, **characterized in that** the cross-section set orthogonally to the central axis (6) is conformed in a noncircular and preferably in an oval or elliptical shape.

6. Intramedullary nail (1) according to one of the claims from 1 to 5, **characterized in that** it presents a longitudinal borehole (7) coaxial with the central axis (6).

7. Intramedullary nail (1) according to one of the claims from 1 to 6, **characterized in that** the proximal extremity (2) is conformed as a straight section (8) of a length P ≤ L.

8. Intramedullary nail (1) according to claim 7, **characterized in that** the length P of the proximal extremity (2) is in the range from 1/6 L to 1/3 L.

9. Intramedullary nail (1) according to one of the claims from 1 to 8, **characterized in that** in the range of the proximal extremity (2), there is at least one locking hole (9) extending across the central axis (6).

10. Intramedullary nail (1) according to one of the claims from 1 to 9, **characterized in that** in the range of the distal extremity (3), there is at least one locking hole (10) extending across the central axis (6).

11. Intramedullary nail (1) according one of the claims from 1 to 10, **characterized in that** the radius of curvature R of the curved section (4) is in the range from 350 to 1200 mm, preferably in the range from 400 to 1100 mm.

12. Intramedullary nail (1) according to one of the claims from 1 to 11, **characterized in that** the ratio L/R is in the range from 0.3 to 0.7, preferably in the range from 0.4 to 0.6.

13. Intramedullary nail (1) according to one of the claims from 1 to 12, **characterized in that** in the range of the distal extremity (3) there are two locking holes (10) extending across the central axis (6).

14. Intramedullary nail (1) according to claim 13, **characterized in that** the two locking holes (10) extending across the central axis (6) enclose an angle of from 45° to 90° between them.

15. Intramedullary nail (1) according to one of the claims from 1 to 14, **characterized in that** the distal extremity (3) presents three locking holes (10, 12, 10), where the central locking hole (12) presents a different distance to the other two locking holes (10).

## Revendications

1. Clou intramédullaire (1), en particulier pour le tibia, comprenant une extrémité proximale (2), une extrémité distale (3) appropriée à être insérée dans la cavité médullaire et un axe central (6), lequel clou présente
A) une longueur totale L dans la gamme 200 à 500 mm ; et
B) un segment courbé (4) de longueur G ≤ L ;
**caractérisé en ce que**
C) le segment courbé (4) de longueur G présente un rayon de courbure R dans la gamme de 300 à 1 300 mm ;
D) le rapport UR est dans la gamme de 0,2 à 0,8 ; et
E) l'extrémité distale (3) est un segment droit (5) de longueur « l » ≤ L.

2. Clou intramédullaire (1) selon la revendication 1, **caractérisé en ce que** la longueur « l » est dans la gamme de 0,20 L à 0,55 L.

3. Clou intramédullaire (1) selon la revendication 2, **caractérisé en ce que** la longueur « l » est dans la gamme de 0,25 L à 0,50 L.

4. Clou intramédullaire (1) selon la revendication 2 ou 3, **caractérisé en ce que** les tangentes au niveau des deux extrémités du segment courbé (4) forment un angle alpha dans la gamme de 7° à 12°, de préférence de 8° à 10°.

5. Clou intramédullaire (1) selon l'une quelconque des revendications 1 à 4, **caractérisé en ce que** la coupe droite orthogonale à l'axe central (6) présente une forme non-ronde et de préférence ovale ou elliptique.

6. Clou intramédullaire (1) selon l'une quelconque des revendications 1 à 5, **caractérisé en ce qu'**il présente un alésage longitudinal (7) coaxial à l'axe central (6).

7. Clou intramédullaire (1) selon l'une quelconque des revendications 1 à 6, **caractérisé en ce que** l'extrémité proximale (2) présente une forme de segment droit (8) de longueur P ≤ L.

8. Clou intramédullaire (1) selon la revendication 7, **caractérisé en ce que** la longueur P de l'extrémité proximale (2) est dans la gamme de 1/6 L à 1/3 L.

9. Clou intramédullaire (1) selon l'une quelconque des revendications 1 à 8, **caractérisé en ce qu'**au moins un trou de verrouillage (9) s'étendant transversalement à l'axe central (6) est présent dans la zone de l'extrémité proximale (2).

10. Clou intramédullaire (1) selon l'une quelconque des revendications 1 à 9, **caractérisé en ce qu'**au moins un trou de verrouillage (10) s'étendant transversalement à l'axe central (6) est présent dans la zone de l'extrémité distale (3).

11. Clou intramédullaire (1) selon l'une quelconque des revendications 1 à 10, **caractérisé en ce que** le rayon de courbure R du segment courbé (4) est dans la gamme de 350 à 1 200 mm, de préférence dans la gamme de 400 à 1 100 mm.

12. Clou intramédullaire (1) selon l'une quelconque des revendications 1 à 11, **caractérisé en ce que** le rapport L/R est dans la gamme de 0,3 à 0,7, de préférence de 0,4 à 0,6.

13. Clou intramédullaire (1) selon l'une quelconque des revendications 1 à 12, **caractérisé en ce que** deux trous de verrouillage (10) s'étendant transversalement à l'axe central (6) sont présents dans la zone de l'extrémité distale (3).

14. Clou intramédullaire (1) selon la revendication 13, **caractérisé en ce que** les deux trous de verrouillage (10) s'étendant transversalement à l'axe central (6) forment un angle de 45° à 90°.

15. Clou intramédullaire (1) selon l'une quelconque des revendications 1 à 14, **caractérisé en ce que** l'extrémité distale (3) présente trois trous de verrouillage (10, 12, 10), dans lequel le trou de verrouillage central (12) se trouve à une distance différente par rapport aux deux autres trous de verrouillage (10).
